# EUROPÄISCHE PATENTSCHRIFT

(11) **EP 0 596 314 B1**
(45) Veröffentlichungstag und Bekanntmachung des Hinweises auf die Patenterteilung: **15.12.1999**
(21) Anmeldenummer: 93116840.5
(22) Anmeldetag: 19.10.1993
(51) Int. Cl.: A61M 3/02

(54) **Ophthalmologisches Aspirations- und Irrigationssystem**
Ophthalmological aspiration and irrigation device
Dispositif d'aspiration et d'irrigation ophtalmologique

(30) Priorität: 06.11.1992 CH 344892; 23.09.1993 CH 287293
(43) Veröffentlichungstag der Anmeldung: 11.05.1994
(73) Patentinhaber: GRIESHABER & CO. AG, CH-8203 Schaffhausen (CH)
(72) Erfinder: Grieshaber, Hans R., CH-8200 Schaffhausen (CH); Demmerle, Rudolf, CH-8200 Schaffhausen (CH); Vogel, Urs, CH-8200 Schaffhausen (CH)
(74) Vertreter: Althoff, Gerhard

(56) Entgegenhaltungen:
- WO-A-87/00759
- WO-A-89/09629
- FR-A- 2 627 707
- US-A- 3 799 702
- US-A- 4 038 980
- US-A- 4 900 301

## Beschreibung

Die Erfindung bezieht sich auf ein Aspirations- und Irrigationssystem zur Aufrechterhaltung des intraokularen Druckes während des operativen Eingriffes am Auge eines Lebewesens, welches System eine mit einer ersten Versorgungsleitung verbundene Druckeinheit zum Zuführen eines gasförmigen Mediums, eine mit einem ersten Behälter und zugeordneter Tropfvorrichtung versehene und zum Zuführen eines flüssigen Mediums über eine zweite Versorgungsleitung mit einer nachgeschalteten Irrigationseinheit verbundene Infusionseinheit sowie eine Aspirationseinheit mit zugeordneter Pumpe umfasst, welche zum Absaugen des jeweils über die entsprechende Versorgungsleitung dem Auge zugeführten gasförmigen oder flüssigen Mediums zusammen mit den während des Eingriffes anfallenden Gewebeteilchen über eine an die Aspirationseinheit angeschlossene Entsorgungsleitung mit dem Auge verbunden ist.

Aus der US-A 4,935,005 ist ein mit einem chirurgischen Instrument in Wirkverbindung stehendes ophthalmologisches Aspirations- und Irrigationssystem bekannt, bei welchem das Instrument einerseits über eine Irrigationsleitung unter Zwischenschaltung eines ersten Schliessventils und eines Kontrollventils mit einem mit einer Spülflüssigkeit gefüllten Behälter und andererseits über eine Aspirationsleitung unter Zwischenschaltung eines Anschlussstücks mit der peristaltisch arbeitenden Pumpe in Verbindung steht. Das mit mehreren Stutzen versehene Anschlussstück ist dabei über eine erste Leitung unter Zwischenschaltung eines Filters mit einem druckempfindlichen Transducer sowie über eine zweite Leitung unter Zwischenschaltung eines weiteren Ventils an die Irrigationsleitung und über die dritte Leitung an dem Instrument angeschlossen sowie über eine weitere Leitung mit der Pumpe verbunden. Eine während des operativen Eingriffs erforderliche, rasch und exakt durchführbare Regulierung des intraokularen Drucks (IOP) ist bei diesem System nicht möglich und es sind zudem keine Mittel vorgesehen, welche eine wahlweise mit dem gasförmigen oder mit dem flüssigen Medium durchführbare Regulierung des intraokularen Drucks ermöglichen.

Ein weiteres ophthalmologisches Irrigations- und Aspirationssystem ist aus der US-A 4,900,301 bekannt, bei welchem System ein Behälter mit Infusionslösung zusammen mit einer Tropfvorrichtung als eine Einheit an einem Ständer angehängt ist und die mit dem Druck in direkter Verbindung stehende Niveauhöhe des Infusionsbehälters von der jeweiligen Augenhöhe des Patienten abhängig ist und durch eine manuelle Höhenverstellung reguliert werden muss. Eine rasch und exakt durchführbare Regulierung des intraokularen Drucks (IOP) ist auch bei diesem System nicht möglich.

Bei den bekannten Systemen wird der intraokulare Druck (IOP) durch die Infusionshöhe des Behälters beziehungsweise durch die Infusionshöhe der Tropfvorrichtung bestimmt. Sobald bei den bekannten Systemen aus bestimmten Gründen, zum Beispiel aufgrund einer auftretenden Blutung, insbesondere während des operativen Eingriffs, der intraokulare Druck (IOP) geändert werden muss, kann dies ausschliesslich durch eine relativ zeitaufwendige und gefühlsmässige Höhenverstellung des Behälters beziehungsweise der Tropfvorrichtung erreicht werden.

Der Erfindung liegt die Aufgabe zugrunde, ein Irrigationsund Aspirationssystem der eingangs genannten Gattung dahingehend zu verbessern und so auszubilden, dass der intraokulare Druck (IOP) während des operativen Eingriffs ohne zusätzliche Hilfsmittel und zeitaufwendige Handgriffe wahlweise mit dem gasförmigen oder flüssigen Medium regulier- und exakt einstellbar ist.

Die Aufgabe wird erfindungsgemäss dadurch gelöst, dass die Infusionseinheit zusätzlich mit einem zwischen der Tropfvorrichtung und der das flüssige Medium der Irrigationseinheit zuführenden zweiten Versorgungsleitung angeordneten zweiten Behälter versehen ist, welcher über eine daran angeschlossene dritte Versorgungsleitung mit der mit mindestens einer mit einem Regelventil wirkverbundenen Druckquelle versehenen Druckeinheit oder mit der davon mit dem gasförmigen Medium beaufschlagten ersten Versorgungsleitung verbunden ist, wobei der erforderliche intraokulare Druck in dem Auge über ein die einzelnen Versorgungsleitungen miteinander verbindendes umschaltbares Mehrwegeventil wahlweise mit dem gasförmigen oder flüssigen Medium regulierbar ist.

Mit dem erfindungsgemässen Aspirations- und Irrigationssystem besteht nunmehr die Möglichkeit, dass einerseits die zu infusierende Flüssigkeitssäule oder andererseits das zu infusierende gasförmige Medium mit exakt zu dosierendem Druck beaufschlagbar ist. Der Druck wird vorzugsweise von einem zugeordneten Messgerät erfasst und an einem Bildfeld des Gerätes angezeigt und kann durch einen an der Druckeinheit vorgesehenen Knopf von dem Augenchirurgen oder Ophthalmologen problemlos und schnell eingestellt werden.

Weitere Merkmale der Erfindung ergeben sich aus der folgenden Beschreibung in Verbindung mit der Zeichnung und den Patentansprüchen.

Die Erfindung wird nachstehend anhand der Zeichnung beschrieben. Es zeigt:
- Fig.1: eine in perspektivischer Ansicht dargestellte ophthalmologische Einrichtung sowie ein damit in Verbindung stehendes, schematisch dargestelltes Aspirations- und Irrigationssystem, und
- Fig.2: das als Blockschaltbild dargestellte Aspirations- und Irrigationssystem für die Einrichtung gemäss Fig.1.

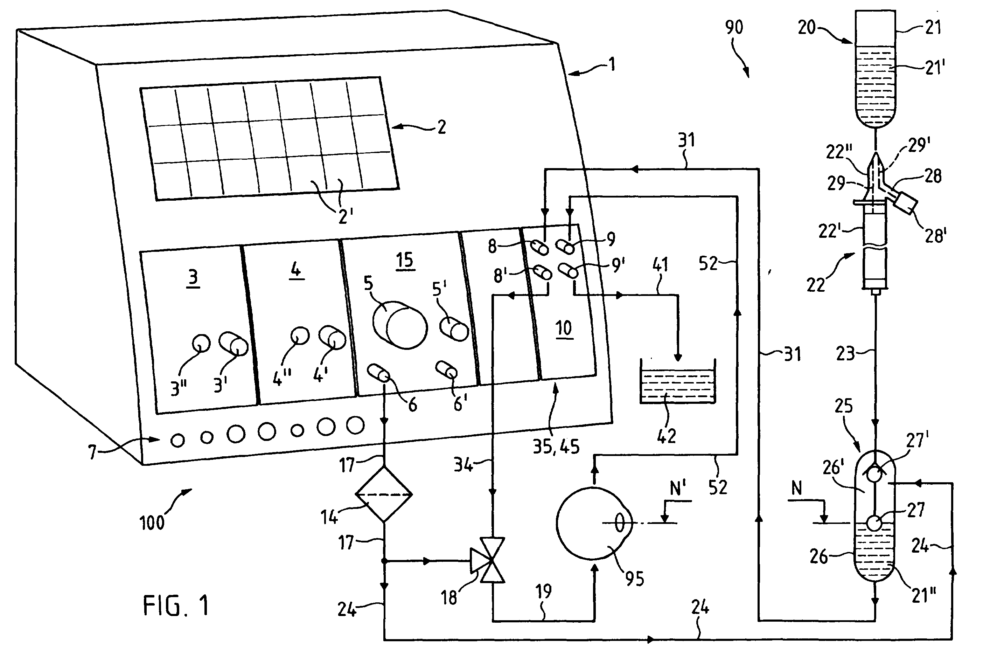

Fig.1 zeigt eine in perspektivischer Ansicht dargestellte und in der Gesamtheit mit 100 bezeichnete ophthalmologische Einrichtung zur Verwendung bei mikrochirurgischen Eingriffen am Auge 95 eines Lebewesens.

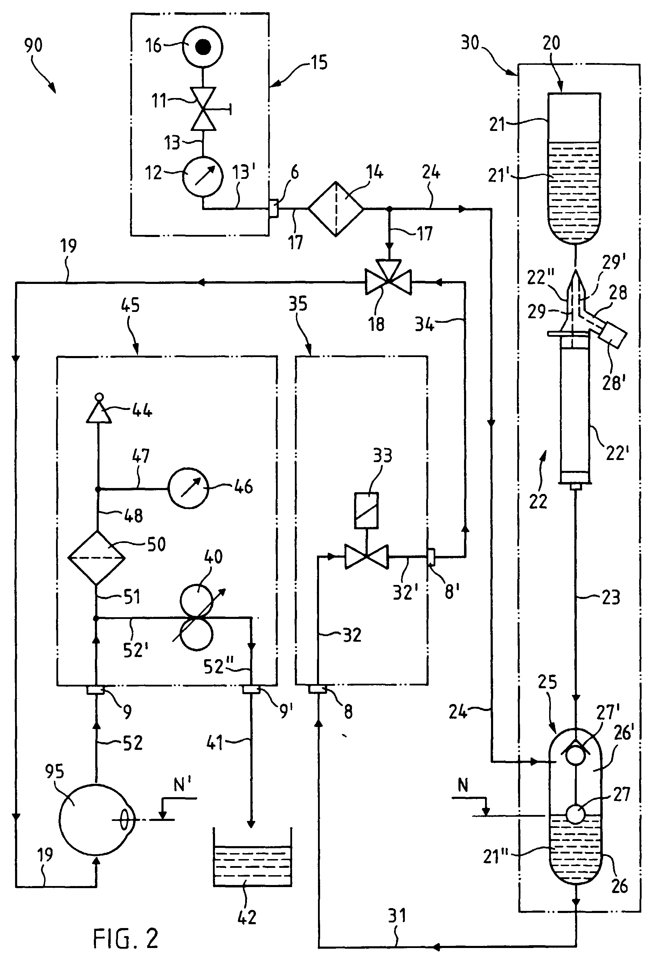

Die Einrichtung 100 umfasst mehrere in einem Gehäuse 1 angeordnete und für den jeweiligen operativen Eingriff ausgebildete Funktionseinheiten 3, 4, 15, 35 und 45. Die einen Funktionseinheiten 15, 35 und 45 stehen, wie in Fig.1 schematisch dargestellt, über entsprechende Versorgungs- und Entsorgungsleitungen mit einem in der Gesamtheit mit 90 bezeichneten Aspirations- und Irrigationssystem in Verbindung. Der spezielle Aufbau des Aspirations- und Irrigationssystems 90 wird später in Verbindung mit Fig.2 im einzelnen beschrieben. Die Funktionseinheiten 3, 4, 15, 35 und 45 sind als auswechselbare, beispielsweise von der Frontseite in das Gehäuse 1 einführbare Einschübe ausgebildet. Weiterhin erkennt man in Fig.1 eine an der Stirnseite des Gehäuses 1 mit mehreren, nicht bezeichneten Anschlüssen versehene Steckerleiste 7, welche zum Anschluss zusätzlicher, nicht näher dargestellter Operationsinstrumente oder dergleichen dient.

Die beiden ersten Funktionseinheiten 3 und 4 sind mit nicht dargestellten Mitteln versehen, mittels welcher beim operativen Eingriff der Augeninnenraum beleuchtet werden kann. Hierzu wird ein nicht dargestelltes, an dem einen Ende mit einer Lichtquelle und an dem anderen Ende mit einem Anschlussteil versehenes Lichtleiterkabel an die entsprechend ausgebildeten Buchsen 3'' und/oder 4'' der Funktionseinheiten 3 und/oder 4 angeschlossen. Die für den operativen Eingriff erforderliche Lichtstärke ist über einen Regler 3' und/oder 4' stufenlos einstell- und regulierbar.

Weiterhin erkennt man in Fig.1 ein an der Frontseite des Gehäuses 1 oberhalb der Funktionseinheiten angeordnetes Bildfeld 2, welches beispielsweise in einzelne Anzeige- und Bedienungsfelder 2' mit LCD-Anzeige (Liquid Crystal Display) unterteilt ist. Das einzelne Anzeige- und Bedienungsfeld 2' dient als programmabhängig gesteuerter Informationsaustausch für den Benutzer der gesamten Einrichtung 100. Die einzelnen Anzeige- und Bedienungsfelder 2' sind durch geringfügigen Fingerdruck für die jeweilige Programmwahl betätigbar, wobei die angewählte Anzeige oder angewählten Anzeigen in bezug auf die nicht betätigten Anzeigen dann beleuchtet sind.

In der nachstehenden Beschreibung wird die erste Funktionseinheit 15 als Druckeinheit 15, die mit einer Infusionseinheit 30 in Verbindung stehende zweite Funktionseinheit 35 als Irrigationseinheit 35 und die dritte Funktionseinheit 45 als Aspirationseinheit 45 bezeichnet.

Die Druckeinheit 15 hat mindestens einen Stutzen 6 zum Anschliessen einer ersten Leitung 17 sowie einen Einstellknopf 5 zum Regulieren, vorzugsweise zum stufenlosen Regulieren der beim operativen Eingriff zur Aufrechterhaltung des intraokularen Druckes erforderlichen Druckluftzufuhr. Der Druckeinheit 15 ist eine nicht näher dargestellte, vorzugsweise im Gehäuse 1 integrierte Visko-Injektionseinrichtung zugeordnet, welche mit einem Stutzen 6' zum Anschliessen einer nicht dargestellten Leitung in Verbindung steht und zur Regulierung einen Einstellknopf 5' aufweist.

Die einzelnen, in Fig.1 nicht näher dargestellten Funktionselemente der Irrigationseinheit 35 und der Aspirationseinheit 45 stehen über einen als Schlauchträger ausgebildeten Einschub 10 miteinander in Wirkverbindung. An der Stirnseite des kassettenförmig ausgebideten Einschubes 10 sind im Abstand zueinander angeordnete Anschlussstutzen 8,8' und 9,9' vorgesehen. Die Funktionselemente der beiden Einheiten 35 und 45 werden in Verbindung mit Fig.2 noch im einzelnen beschrieben.

Die Druckeinheit 15, die Irrigationseinheit 35 und die Aspirationseinheit 45 der ophthalmologischen Einrichtung 100 stehen über entsprechend angeschlossene Versorgungs- und Entsorgungsleitungen einerseits mit einer im wesentlichen ein erstes sowie ein zweites Infusionsgerät 20, 25 umfassende Infusionseinheit 30 und andererseits mit einem nicht dargestellten Infusionselement in Verbindung.

Das erste Infusionsgerät 20 hat einen mit einer Salzlösung 21' gefüllten ersten Behälter 21, welcher über eine Leitung 23 unter Zwischenschaltung einer Tropfvorrichtung 22 mit einem zweiten Behälter 26 des zweiten Infusionsgerätes 25 verbunden ist. In dem zweiten Behälter 26 des zweiten Infusionsgerätes 25 ist ein Schwimmerventil 27 angeordnet, mit welchem das Niveau N der über die Leitung 23 zugeführten Salzlösung 21'' überwacht und mit nicht dargestellten Mitteln reguliert wird. Das Niveau N der vom ersten Behälter 21 zugeführten Salzlösung 21'' im zweiten Behälter 26 kann über das mit nicht dargestellten Regelelementen in Wirkverbindung stehende Schwimmerventil 27 durch Zuführung der Salzlösung 21' aus dem ersten Behälter 21 konstant gehalten werden. Die Höhe des Niveaus N der Salzlösung 21'' im zweiten Behälter 26 ist somit auf die Niveauhöhe N' des zu operierenden Auges 95 eines auf einem nicht dargestellten Operationstisch liegenden Patienten einstellbar. Wesentlich ist hierbei, dass während des operativen Eingriffes die beiden Niveauhöhen N und N' zumindest annähernd gleich sind. Das zweite Infusionsgerät 25 ist über eine unten am zweiten Behälter 26 befestigte zweite Leitung 31 an den Stutzen 8 der Irrigationseinheit 35 angeschlossen.

Im oberen Bereich des zweiten Behälters 26 ist eine bis in den Innenraum 26' reichende dritte Leitung 24 angeordnet, welche mit der am Stutzen 6 der Druckeinheit 15 angeschlossenen ersten Leitung 17 derart verbunden ist, dass der oberhalb der Salzlösung 21'' befindliche Behälterraum 26' mit Druckluft beaufschlagbar ist. Von dem zweiten Behälter 26 wird die Salzlösung 21'' über die am ersten Stutzen 8 der Irrigationseinheit 35 angeschlossene zweite Leitung 31 sowie über ein am zweiten Stutzen 8' der Irrigationseinheit 35 angeschlossenes erstes Leitungsteilstück 34 und von dort über ein Mehrwegeventil 18 sowie über ein daran angeschlossenes zweites Leitungsteilstück 19 dem zu operierenden Auge 95 zugeführt.

Die erste Leitung 17 ist mit dem einen Ende an die Druckeinheit 15 und unter Zwischenschaltung eines Filters 14 mit dem anderen Ende an das Mehrwegeventil 18 angeschlossen, wobei das Mehrwegeventil 18 über die Leitung 34 an die Irrigationseinheit 35 angeschlossen ist. Durch diese Leitungsverbindung wird erreicht, dass mit einer entsprechenden Umschaltung des Mehrwegeventils 18 über den Filter 14 und die beiden Leitungen 17 und 19 dem Auge 95 keimfreie Druckluft oder aber von der Irrigationseinheit 35 über die beiden Leitungsteilstücke 34 und 19 flüssiges Medium, vorzugsweise die Salzlösung 21'' aus dem zweiten Behälter 26 zuführbar ist.

Die im zweiten Behälter 26 befindliche Salzlösung 21'' wird über die erste Leitung 17, den Filter 14 sowie über die dritte Leitung 24 mit keimfreier Druckluft beaufschlagt, wobei der erforderliche Druck an der Druckeinheit 15 einstellbar ist. Die Salzlösung 21'' kann somit mit vorbestimmtem, beispielsweise stufenlos einstellbarem Druck dem zu operierenden Auge 95 zur Aufrechterhaltung des intraokularen Druckes (IOP) zugeführt werden.

Bei einer nicht dargestellten Variante besteht auch die Möglichkeit, dass die mit dem Behälterraum 26' des zweiten Behälters 26 in Verbindung stehende dritte Leitung 24 unter Zwischenschaltung des Filters 14 an einen nicht dargestellten Stutzen der Druckeinheit 15 angeschlossen ist, so dass der zweite Behälter 26 direkt mit keimfreier Druckluft beaufschlagbar ist.

Die während des operativen Eingriffs im Auge 95 anfallenden Gewebeteilchen werden, wie in Fig.1 schematisch dargestellt, über eine am Auge 95 sowie am ersten Stutzen 9 der Aspirationseinheit 45 angeschlossene Leitung 52 abgesaugt und über eine weitere am Stutzen 9' angeschlossene Leitung 41 einem Abfallbehälter 42 zugeführt. Der Abfallbehälter 42 kann auch in nicht näher dargestellter Weise im Gehäuse 1 (Fig. 1) der Einrichtung 100 angeordnet werden.

In Fig.2 ist das Aspirations- und Irrigationssystem 90 als Blockschaltbild dargestellt, und man erkennt die Druckeinheit 15, die Infusionseinheit 30, die Irrigationseinheit 35 sowie die Aspirationseinheit 45. Die Einheiten 15, 30, 35 und 45 mit den einzelnen Funktionselementen werden nachstehend beschrieben.

Die Druckeinheit 15 hat eine Druckquelle 16, ein Regelventil 11 sowie ein Druckmessgerät 12, wobei die einzelnen Teile über ein erstes Leitungsteil 13 miteinander und über ein zweites Leitungsteil 13 mit dem Anschlussstutzen 6 der Druckeinheit 15 verbunden sind. Von dem Druckmessgerät 12 wird der mit dem Regelventil 11 eingestellte Druck erfasst und der Wert an einem der Bedienungsfelder 2' (Display) der Einrichtung 100 (Fig.1) angezeigt, wobei der Druck entsprechend den Erfordernissen mit dem Knopf 5 reguliert, beispielsweise stufenlos reguliert werden kann.

Die in Verbindung mit Fig.1 bereits erwähnte Infusionseinheit 30 umfasst den ersten Behälter 21 mit der Salzlösung 21', die mit dem ersten Behälter 21 in Verbindung stehende Tropfvorrichtung 22 und den über die Leitung 23 damit in Verbindung stehenden und mit einem Schwimmerventil 27 versehenen zweiten Behälter 26.

Die schematisch dargestellte Tropfvorrichtung 22 hat ein beispielsweise direkt am ersten Behälter 21 angeschlossenes, rohrförmiges Anschlussstück 22'' sowie einen daran angeordneten Auffangbehälter 22', welcher über die daran angeschlossene Leitung 23 mit dem zweiten Behälter 26 verbunden ist. Seitlich an dem Anschlussstück 22'' ist ein Stutzen 28 angeordnet, welcher zur Aufnahme eines Filters 28' ausgebildet ist. Der Auffangbehälter 22' und der erste Behälter 21 stehen über einen das Anschlussstück 22'' in axialer Richtung durchdringenden ersten Kanal 29 miteinander in Verbindung. Der Stutzen 28 ist mit einem zweiten Kanal 29' versehen, welcher sich durch das Anschlussstück 22'' bis in den ersten Behälter 21 erstreckt, so dass beim Ablaufen der Salzlösung 21' in den zweiten Behälter 26 gleichzeitig die über den Filter angesaugte, keimfreie Luft in den ersten Behälter 21 gelangt.

Beim Absinken des Flüssigkeits-Niveaus N im zweiten Behälter 26 entsteht gleichzeitig in der Tropfvorrichtung 22 ein Unterdruck, wobei beim Erreichen eines vorbestimmten MinimalNiveaus N im zweiten Behälter 26 von dem Schwimmerventil 27 ein Ventil 27' geöffnet wird. Bei geöffnetem Ventil 27' fliesst dann die Flüssigkeit 21' von dem ersten Behälter 21, unter gleichzeitigem Ansaugen keimfreier Luft über den Filter 28' und den zweiten Kanal 29' in den ersten Behälter 21, durch den ersten Kanal 29 in den Auffangbehälter 22' der Tropfvorrichtung 22 und von dort über die Leitung 23 in den zweiten Behälter 26 ab. Sobald die Flüssigkeit 21'' im zweiten Behälter 26 das vorbestimmte Flüssigkeits-Niveau N wieder erreicht hat, wird das Ventil 27' wieder geschlossen. Das vorstehend beschriebene Nachfliessen des flüssigen Mediums 21' zum zweiten Behälter 26 sowie das Öffnen und Schliessen des Ventils 27' erfolgt automatisch unter absolut sterilen Verhältnissen.

Die Irrigationseinheit 35 umfasst im wesentlichen ein mit nicht dargestellten Mitteln ansteuerbares Unterbrecherorgan 33, welches über Leitungen 32,32' mit den beiden Anschlussstutzen 8,8' verbunden ist. Das Unterbrecherorgan 33 ist vorzugsweise ein ansteuerbares Magnetventil 33, welches ein nicht dargestelltes Quetschelement hat, mittels welchem die elastische Leitung 32,32' je nach Ansteuerung abgeklemmt oder geöffnet die Zufuhr des flüssigen Mediums 21'' zum Auge 95 durch das Magnetventil 33 unterbrochen wird. Die Irrigationseinheit 35 steht weiterhin über das am Stutzen 8' angeschlossene erste Leitungsteilstück 34 mit dem Mehrwegeventil 18 und über das am Mehrwegeventil 18 angeschlossene zweite Leitungsteilstück 19 mit dem Auge 95 in Verbindung. Das Auge 95 ist weiterhin über die Leitung 52 mit der Aspirationseinheit 45 verbunden.

Die mit den beiden Anschlüssen 9,9' versehene Aspirationseinheit 45 umfasst eine Pumpe, beispielsweise eine schematisch dargestellte peristaltisch arbeitende Pumpe 40, einen Filter 50, ein Druckmessgerät 46 sowie ein Belüftungsventil 44, wobei die einzelnen Teile 40, 50, 46 und 44 über entsprechende Leitungen 47, 48, 51, 52' und 52'' miteinander verbunden sind. Weiterhin erkennt man die an den Stutzen 9' angeschlossene sowie mit dem Abfallbehälter 42 in Verbindung stehende Leitung 41.

Bei dem vorstehend beschriebenen Aspirations- und Irrigationssystem 90 bilden die beiden Leitungen 17 und 19 eine erste Versorgungsleitung 17;19 mit welcher über den Filter 14 keimfreies, gasförmiges Medium dem zu operierenden Auge 95 zugeführt wird. Die beiden mit der ersten Versorgungsleitung 17;19 verbundenen Leitungen 31 und 34 bilden eine zweite Versorgungsleitung 31;34 mit welcher dem Auge 95 flüssiges Medium zugeführt wird. Die an den Filter 14 sowie an die Leitung 17 angeschlossene Leitung 24 bildet eine dritte Versorgungsleitung 24, über welche die im Behälter 26 befindliche Flüssigkeit 21'' mit dem keimfreien, gasförmigen Medium druckbeaufschlagt wird.

Dem zu operierenden Auge 95 kann somit mit regulierbarem Druck und bei einer ersten Stellung des Mehrwegeventils 18 von der Druckeinheit 15 über die erste Versorgungsleitung 17; 19 keimfreies, gasförmiges Medium zugeführt werden. Bei einer zweiten Stellung des Mehrwegeventils 18 kann dem zu operierenden Auge 95 von dem zweiten Behälter 26 über die zweite Versorgungsleitung 31;34 und dem Mehrwegeventil 18 das keimfreie, flüssige Medium zugeführt werden. Als flüssiges Medium wird beispielsweise eine geeignete Salzlösung 21'' verwendet.

Die vom Auge 95 zur Aspirationseinheit 45 führende Leitung 52 bildet eine erste Entsorgungsleitung und die von der Aspirationseinheit 45 zum Behälter 42 führende Leitung 41 eine zweite Entsorgungsleitung.

An dieser Stelle wird darauf hingewiesen, dass sämtliche Leitungen für die gasförmigen und flüssigen Medien als flexible Schlauchleitungen ausgebildet und vorzugsweise aus transparentem Kunststoff oder dergleichen hergestellt sind. Die beiden Behälter 21 und 26 der Infusionseinheit 30 sowie die komplette Tropfvorrichtung 22 ist ebenfalls aus transparentem Kunststoff hergestellt.

Bei einem bevorzugten Ausführungsbeispiel wird der momentane, intraokulare Druck (IOP) im Auge durch ein Druckmessgerät permanent überwacht und am Bildfeld 2 des Gerätes 100 (Fig.1) angezeigt. In Abhängigkeit des gemessenen Druckes kann der Druck des zuführbaren gasförmigen oder flüssigen Infusionsmediums durch eine entsprechende Bestimmung des gewünschten Druckes am Bildfeld 2 des Gerätes 100 eingestellt (durch geringen Fingerdruck) und somit entweder erhöht oder gesenkt werden.

An dieser Stelle wird darauf hingewiesen, dass die mit der Druckquelle 16, dem Regelventil 11 und dem Druckmessgerät 12 versehene Druckeinheit 15 zudem mit nicht dargestellten Mitteln versehen ist oder in Wirkverbindung steht, anhand welcher der eingestellte Druck mit dem tatsächlich vorhandenen Druck verglichen und an einem der Bedienungsfelder 2' (Display) der Einrichtung 100 angezeigt wird.

Bei dem dargestellten Ausführungsbeispiel sind die einzelnen Funktionseinheiten 3, 4, 15, 35 und 45 in dem Gehäuse 1 der Einrichtung angeordnet und als auswechselbare Einschübe ausgebildet. Es wird darauf hingewiesen, dass die einzelnen Funktionseinheiten jedoch auch jeweils für sich allein in einem Gehäuse angeordnet oder für sich allein aufgestellt werden können.

## Patentansprüche

1. Aspirations- und Irrigationssystem (90) zur Aufrechterhaltung des intraokularen Druckes während des operativen Eingriffes am Auge (95) eines Lebewesens welches System eine mit einer ersten Versorgungsleitung (17;19) verbundene Druckeinheit (15) zum Zuführen eines gasförmigen Mediums, eine mit einem ersten Behälter (21) und zugeordneter Tropfvorrichtung (22) versehene und zum Zuführen eines flüssigen Mediums über eine zweite Versorgungsleitung (31;34) mit einer nachgeschalteten Irrigationseinheit (35) verbundene Infusionseinheit (30) sowie eine Aspirationsheit (45) mit zugeordneter Pumpe (40) umfasst, welche zum Absaugen des jeweils über die entsprechende Versorgungsleitung dem Auge (95) zugeführten gasförmigen oder flüssigen Mediums zusammen mit den während des Eingriffes anfallenden Gewebeteilchen über eine an die Aspirationseinheit angeschlossene Entsorgungsleitung (41;52) mit dem Auge (95) verbunden ist, **dadurch gekennzeichnet**, dass die Infusionseinheit (30) zusätzlich mit einem zwischen der Tropfvorrichtung (22) und der das flüssige Medium der Irrigationseinheit (35) zuführenden zweiten Versorgungsleitung (31) angeordneten zweiten Behälter (26) versehen ist, welcher über eine daran angeschlossene dritte Versorgungsleitung (24) mit der mit mindestens einer mit einem Regelventil (11) wirkverbundenen Druckquelle (16) versehenen Druckeinheit (15) oder mit der davon mit dem gasförmigen Medium beaufschlagten ersten Versorgungsleitung (17;19) verbunden ist, wobei der erforderliche intraokulare Druck in dem Auge (95) über ein die einzelnen Versorgungsleitungen (17;19;24;31;34) miteinander verbindendes umschaltbares Mehrwegeventil (18) wahlweise mit dem gasförmigen oder flüssigen Medium regulierbar ist.

2. Aspirations- und Irrigationssystem nach Anspruch 1, **dadurch gekennzeichnet**, dass über die an die Druckeinheit (15) angeschlossene erste Versorgungsleitung (17;19) sowie unter Zwischenschaltung eines Filters (14) bei einer ersten Stellung des umschaltbaren Mehrwegeventils (18) der intraokulare Druck in dem Auge (95) durch ein keimfreies, gasförmiges Medium regulierbar ist.

3. Aspirations- und Irrigationssystem nach den Ansprüchen 1 und 2, **dadurch gekennzeichnet**, dass das von dem zweiten Behälter (26) zur Regulierung des intraokularen Druckes dem Auge (95) zuführbare flüssige Medium (21'') bei einer zweiten Stellung des umschaltbaren Mehrwegeventils (18) über die dritte Versorgungsleitung (24) mit dem unter Druck stehenden keimfreien gasförmigen Medium beaufschlagbar ist.

4. Aspirations- und Irrigationssystem nach den Ansprüchen 1 bis 3, **dadurch gekennzeichnet**, dass die mit der Druckquelle (16) und dem Regelventil (11) versehene Druckeinheit (15) zusätzlich mit einem Druckmessgerät (12) und mit Steuermitteln versehen ist, anhand welcher der eingestellte Druck mit dem momentanen Druck vergleichbar und in Abhängigkeit des Differenzwertes an dem Regelventil (11) neu einstellbar ist.

5. Aspirations- und Irrigationssystem nach Anspruch 1, **dadurch gekennzeichnet**, dass die mit der Druckeinheit (15) in Verbindung stehende dritte Versorgungsleitung (24) vorzugsweise oberhalb eines maximal möglichen Flüssigkeits-Niveaus (N) an dem zweiten Behälter (26) angeschlossen ist und der zweite Behälter (26) mit einem in Abhängigkeit des vorhandenen Flüssigkeits-Niveaus (N) die Flüssigkeitszufuhr von dem ersten Behälter (21) regulierenden, vorzugsweise automatisch regulierenden Schwimmerventil (27;27') versehen ist.

6. Aspirations- und Irrigationssystem nach Anspruch 1, **dadurch gekennzeichnet**, dass die mit der zweiten Versorgungsleitung (31) in Verbindung stehende Irrigationseinheit (35) mindestens ein als Unterbrecherorgan ausgebildetes Magnetventil (33) und Mittel zur Steuerung desselben aufweist, mittels welchem/welcher die Zufuhr des keimfreien flüssigen Mediums (21'') zu dem Auge (95) einstellbar ist.

7. Aspirations- und Irrigationssystem nach Anspruch 6, **dadurch gekennzeichnet**, dass die zweite Versorgungsleitung (31) aus elastischem Material besteht und das Magnetventil (33) mit einem mit der zweiten Versorgungsleitung (31) in Eingriff bringbaren und die Zufuhr des keimfreien flüssigen Mediums (21'') unterbrechenden Quetschelement versehen ist.

8. Aspirations- und Irrigationssystem nach Anspruch 1, **dadurch gekennzeichnet**, dass die Aspirationseinheit (45) die vorzugsweise peristaltisch arbeitende Pumpe (40) und nachgeschaltet einen Filter (50), ein Messgerät (46) sowie ein Belüftungsventil (44) umfasst sowie mit Mitteln versehen ist, anhand welcher die eingestellte und am Messgerät (46) angezeigte Saugleistung der Pumpe (40) regulierend einstellbar ist.

9. Aspirations- und Irrigationssystem nach Anspruch 1, **dadurch gekennzeichnet**, dass die Druckeinheit (15), die Irrigationseinheit (35) und die Aspirationseinheit (45) in einem Gehäuse (1) einer ophthalmologischen Einrichtung (100) angeordnet und über die einzelnen Versorgungsleitungen für das gasförmige und flüssige Medium mit der Infusionseinheit (30) und über die am Auge (95) angeschlossene Entsorgungsleitung (52;52') mit der Pumpe (40) der Aspirationseinheit (45) verbunden sind.

10. Aspirations- und Irrigationssystem nach Anspruch 9, **dadurch gekennzeichnet**, dass die Einrichtung (100) mit anwählbaren und in Form von als Tastschaltern ausgebildeten Anzeige- und Bedienungsfeldern (2,2') oder eines Einstellknopfs (5) ausgebildeten Steuermitteln versehen ist, anhand welcher die Zufuhr des gasförmigen oder flüssigen und druckbeaufschlagten Mediums zu dem Auge (95) einstell- und regulierbar ist.

11. Aspirations- und Irrigationssystem nach den Ansprüchen 9 und 10, **dadurch gekennzeichnet**, dass der momentane intraokulare Druck im Auge (95) permanent überwacht und an mindestens einem der Anzeige- und Bedienungsfelder (2,2') der Einrichtung (100) angezeigt wird, wobei in Abhängigkeit des angezeigten Wertes der erforderliche Druck des gasförmigen oder flüssigen Mediums über die Druckeinheit (15) einstell- und regulierbar ist.

## Claims

1. Aspiration and irrigation system (30) for maintaining the intraocular pressure whilst carrying out surgery on the eye (95) of a living creature, said system comprising a pressure unit (15) connected to a first supply line (17, 19) for conveying a gaseous medium, an infusion unit (30) provided with a first container (21) and an associated drip device (22) wherein said infusion unit is connected to a following irrigation unit (35) via a second supply line (31; 34) for conveying a fluid medium, and also comprising an aspiration unit (45) having an associated pump (40) which is connected to the eye (95) via a waste removal line (41; 52) attached to the aspiration unit for drawing off the gaseous or fluid medium conveyed via the respective supply line to the eye (95) together with the tissue particles resulting from the surgical operation,
characterised in that the infusion unit (33) is addicionally provided with a second container (26) arranged between the drip device (22) and the second supply line (31) conveying the fluid medium to the irrigation unit (35), said second container being connected via a third supply line (24) attached thereto to the pressure unit (15) comprising at least one pressure source (16) that is operatively linked to a control valve (11) or to the first supply line (17; 19) supplied with the gaseous medium by said pressure unit (15), whereby the intraocular pressure required in the eye (95) is controllable by the gaseous or the fluid medium in selective manner via a switchable multi-way valve (18) connecting the individual supply lines (17; 19; 24; 31; 34) to one another.

2. Aspiration and irrigation system in accordance with Claim 1, characterised in that, in a first position of the switchable multi-way valve (18), the intraocular pressure in the eye (95) is controllable by a germ-free gaseous medium via the first supply line (17; 19) connected to the pressure unit (15) and the intermediary of an in-line filter (14).

3. Aspiration and irrigation system in accordance with Claims 1 and 2, characterised in that, in a second position of the switchable multi-way valve (18), the fluid medium (21'') suppliable from the second container (26) for controlling the intraocular pressure in the eye (95) may be subjected to the pressurised, germ-free gaseous medium via the third supply line (24).

4. Aspiration and irrigation system in accordance with Claims 1 to 3, characterised in that the pressure unit (15) comprising the pressure source (16) and the control valve (11) is also provided with a pressure measuring device (12) and a control means with the aid of which the instantaneous pressure can be compared with the set pressure and can be reset by the control valve (11) in dependence on the difference value.

5. Aspiration and irrigation system in accordance with Claim 1, characterised in that the third supply line (24) connected to the pressure unit (15) is preferably attached to the second container (26) above a maximum possible fluid level (N), and the second container (26) is provided with a float valve (27; 27') which controls, preferably automatically controls, the supply of fluid from the first container (21) in dependence on the existing fluid level (N).

6. Aspiration and irrigation system in accordance with Claim 1, characterised in that the irrigation unit (35) connected to the second supply line (31) comprises at least one magnetic valve (33) in the form of an interrupter member together with means for controlling it, these being used for adjusting the supply of the germ-free fluid medium (21'') to the eye (95).

7. Aspiration and irrigation system in accordance with Claim 6, characterised in that the second supply line (31) consists of resilient material and the magnetic valve (33) is provided with a pinch element which is engageable with the second supply line (31) so as to interrupt the supply of the germ-free fluid medium (21'').

8. Aspiration and irrigation system in accordance with Claim 1, characterised in that the aspiration unit (45) comprises the preferably peristaltic pump (40) together with a filter (50), a measuring device (46) and a breather valve (44) which are connected thereafter, and is also provided with means with the aid of which the suction power of the pump (40) as set and indicated by the measuring device (46) is adjustable in regulatory manner.

9. Aspiration and irrigation system in accordance with Claim 1, characterised in that the pressure unit (15), the irrigation unit (35) and the aspiration unit (45) are arranged in a housing (1) of an ophthalmological apparatus (100) and are connected to the infusion unit (30) via the individual supply lines for the gaseous and fluid medium and to the pump (40) of the aspiration unit (45) via the waste removal line (52; 52') attached to the eye (95).

10. Aspiration and irrigation system in accordance with Claim 9, characterised in that the apparatus (100) is provided with selectable display and operational fields (2, 2') constructed in the form of push-buttons or control means in the form of an adjusting knob (5) with the aid of which the supply of the pressurised, gaseous or fluid medium to the eye (95) is adjustable and controllable.

11. Aspiration and irrigation system in accordance with Claims 9 and 10, characterised in that the instantaneous intraocular pressure in the eye (95) is permanently monitored and indicated on at least one of the display and operational fields (2, 2') in the apparatus (100), whereby the necessary pressure of the gaseous or fluid medium is adjustable and controllable via the pressure unit (15) in dependence on the value displayed.

## Revendications

1. Système d'aspiration et d'irrigation (90) permettant de maintenir la pression intra-oculaire au cours de l'intervention chirurgicale sur l'il (95) d'un être vivant, lequel système comprend une unité de pression (15) reliée à une première conduite d'alimentation (17;19) et permettant d'amener un milieu gazeux, une unité d'infusion (30) équipée d'un premier réservoir (21) et d'un dispositif compte-gouttes coordonné (22), et reliée à une unité d'irrigation (35) intercalée en arrière pour amener un milieu liquide par le biais d'une seconde conduite d'alimentation (31;34), ainsi qu'une unité d'aspiration (45) avec pompe coordonnée (40), laquelle, pour aspirer le milieu gazeux ou liquide amené dans l'il (95) par la conduite d'alimentation correspondante, ainsi que les parties du tissu produites pendant l'intervention, est reliée à l'il (95), par le biais d'une conduite d'élimination (41;52) raccordée à l'unité d'aspiration, **caractérisé en ce que** l'unité d'infusion (30) est de plus équipée d'un second réservoir (26) disposé entre le dispositif compte-gouttes (22) et la seconde conduite d'alimentation (31) amenant le milieu liquide vers l'unité d'irrigation (35), lequel réservoir est relié par le biais d'une troisième conduite d'alimentation (24) s'y raccordant à l'unité de pression (15) équipée d'au moins une source de pression (16) reliée par action coordonnée à une valve de régulation (11) ou à la première conduite d'alimentation (17;19) alimentée avec le milieu gazeux, la pression intraoculaire nécessaire dans l'il (95) pouvant être régulée au choix par le milieu gazeux ou liquide par l'intermédiaire d'une valve multivoie (18) commutable et reliant ensemble les différentes conduites d'alimentation (17;19;24;31;34).

2. Système d'aspiration et d'irrigation selon la revendication 1, **caractérisé on ce que** la pression intraoculaire de l'il (95) peut être régulée par un milieu gazeux exempt de germes par le biais de la première conduite d'alimentation (17;19) raccordée à l'unité de pression (15) et par intercalation d'un filtre (14) selon une première position de la valve multivoie commutable (18).

3. Système d'aspiration et d'irrigation selon les revendications 1 et 2, **caractérisé en ce que** le milieu liquide (21'') pouvant être amené dans l' il (95) depuis le second réservoir (26) pour réguler la pression intra-oculaire peut être alimenté, selon une seconde position de la valve multivoie commutable (18), par le milieu gazeux exempt de germes sous pression par le biais de la troisième conduite d'alimentation (24).

4. Système d'aspiration et d'irrigation selon les revendications 1 à 3, **caractérisé en ce que** l'unité de pression (15) équipée de la source de pression (16) et de la valve de régulation (11) est de plus équipée d'un manomètre (12) et d'éléments de commande, au moyen desquels la pression réglée peut être comparée à la pression instantanée et à nouveau réglée au niveau de la valve de régulation (11) en fonction de la valeur de différence.

5. Système d'aspiration et d'irrigation selon la revendication 1, **caractérisé en ce que** la troisième conduite d'alimentation (24) en relation avec l'unité de pression (15) est de préférence raccordée au second réservoir (26) audessus d'un niveau de liquide maximum possible (N), et en ce que le second réservoir (26) est pourvu d'une valve à flotteur (27;27') régulant, de préférence automatiquement, l'alimentation en liquide provenant du premier réservoir (21) en fonction du niveau de liquide (N) existant.

6. Système d'aspiration et d'irrigation selon la revendication 1, **caractérisé en ce que** l'unité d'irrigation (35) en relation avec la seconde conduite d'alimentation (31) comprend au moins une électrovalve (33) conçue en tant qu'organe d'interruption et des moyens pour commander celle-ci, au moyen duquel/desquels l'alimentation de l'il (95) en milieu liquide exempt de germes (21'') peut être réglée.

7. Système d'aspiration et d'irrigation selon la revendication 6, **caractérisé en ce que** la seconde conduite d'alimentation (31) se compose d'un matériau élastique et en ce que l'électrovalve (33) est munie d'un élément de pincement pouvant être amené en prise avec la seconde conduite d'alimentation (31) et interrompant l'alimentation en milieu liquide exempt de germes (21'').

8. Système d'aspiration et d'irrigation selon la revendication 1, **caractérisé en ce que** l'unité d'aspiration (45) comprend la pompe de préférence péristaltique (40) et, intercalé après, un filtre (50), un appareil de mesure (46) et une valve de ventilation (44), et est munie de moyens au moyen desquels la puissance d'aspiration de la pompe (40) réglée et affichée sur l'appareil de mesure (46) est réglable par régulation.

9. Système d'aspiration et d'irrigation selon la revendication 1, **caractérisé en ce que** l'unité de pression (15), l'unité d'irrigation (35) et l'unité d'aspiration (45) sont disposées dans un boîtier (1) d'un dispositif ophtalmologique (100) et sont reliées à l'unité d'infusion (30) par le biais des différentes conduites d'alimentation en milieu gazeux ou liquide et à la pompe (40) de l'unité d'aspiration (45) par le biais de la conduite d'élimination (52;52') raccordée à l'il (95).

10. Système d'aspiration et d'irrigation selon la revendication 9, **caractérisé en ce que** le dispositif (100) est équipé de champs d'affichage et de commande (2,2') pouvant être sélectionnés et se présentant sous forme de touches commutables, ou d'éléments de commande conçus sous la forme d'un bouton de réglage (5), au moyen desquels l'amenée du milieu liquide ou gazeux chargé en pression vers l'il (95) est réglable et régulable.

11. Système d'aspiration et d'irrigation selon les revendications 9 et 10, **caractérisé en ce que** la pression intra-oculaire instantanée de l'il (95) est surveillée en permanence et affichée sur au moins un des champs d'affichage et de commande (2,2') du dispositif (100), suite à quoi, en fonction de la valeur affichée, la pression nécessaire du milieu gazeux ou liquide peut être réglée et régulée par le biais de l'unité de pression (15).
